# EUROPÄISCHE PATENTSCHRIFT

(11) **EP 1 222 909 B1**
(45) Veröffentlichungstag und Bekanntmachung des Hinweises auf die Patenterteilung: **31.01.2007**
(21) Anmeldenummer: 01130901.0
(22) Anmeldetag: 27.12.2001
(51) Int. Cl.: A61K 6/083, C04B 28/28

(54) **Wasserbasierender Zement**
Hydraulic cement
Ciment hydraulique

(30) Priorität: 14.01.2001 DE 10101301
(43) Veröffentlichungstag der Anmeldung: 17.07.2002
(73) Patentinhaber: VOCO GmbH, 27472 Cuxhaven (DE)
(72) Erfinder: van Eikeren, Andreas, Dr., 27474 Cuxhaven (DE); Plaumann, Manfred Thomas, 27476 Cuxhaven (DE)

(56) Entgegenhaltungen:
- EP-A- 0 922 683
- WO-A-00/45867
- DE-A- 3 610 844
- DE-A- 3 941 629
- DE-A- 19 536 018
- DE-A- 19 904 816
- GB-A- 2 108 132
- PATENT ABSTRACTS OF JAPAN vol. 016, no. 273 (C-0953), 18. Juni 1992 (1992-06-18) & JP 04 069310 A (MITSUBISHI MATERIALS CORP), 4. März 1992 (1992-03-04)

## Beschreibung

Die vorliegende Erfindung betrifft einen wasserbasierenden Glasionomer-Zement, insbesondere zur Verwendung in der Zahnheilkunde und Medizin, wobei dem Zement 0,01 bis 10 Gew.% einer bei Raumtemperatur flüssigen aliphatischen Monocarbonsäure mit mehr als 5 C-atomen oder einer bei Raumtemperatur flüssigen Mischung aus aliphatischen Monocarbonsäuren mit mehr als 5 C-atomen zugesetzt ist.

Glasionomerzemente sind seit 1969 durch A. D. Wilson und B. E. Kent, DE 2061513, bekannt. Der Glasionomerzement ist ein Zement, der in einer Säure-Base-Reaktion aushärtet. Die drei wesentlichen Bestandteile des Glasionomerzementes sind das Alumino(fluoro)silikatglas als Base, ein Polyelektrolyt, meist Polyacrylsäure, als Säure und Wasser. Weitere übliche Bestandteile der Glasionomerzemente sind chelatbildende Säuren, wie z.B. Weinsäure zur Verbesserung des Abbindeverhaltens, und weitere spezielle Zusätze wie beispielsweise Farbstoffe, Pigmente oder Silberpartikel. Der Glasionomerzement bindet unter Bildung eines Gels ab, als Ergebnis einer Säure-Base-Reaktion, wobei der Glasfüllstoff an der Abbindereaktion teilnimmt. Die Bestandteile verteilen sich dabei auf die anzumischenden Pulver- und Flüssigkeitskomponenten. Da die Zusammensetzungen der Gläser und die der Polyelektrolyte jeweils stark variieren können, ergeben sich vielfältige Möglichkeiten in der Zementbeschaffenheit.

Glasionomerzemente haften an der Zahnhartsubstanz und sind biologisch gut verträglich. Aufgrund der enthaltenden Fluoridverbindungen sind Glasionomerzemente in der Lage durch Fluoridabgabe im Mundmilieu eine kariesvorbeugende Wirkung zu erzielen. Glasionomerzemente schrumpfen bei der Aushärtung nicht und haben im Vergleich mit der Zahnhartsubstanz ein ähnliches thermisches Ausdehnungsverhalten. Weiterhin ist deren Handhabung im Vergleich zu dentalen Compositematerialien relativ einfach. Umständliche Arbeitsschritte wie Trockenlegung des Behandlungsgebietes, Ätzen und Bonden der Zahnhartsubstanz und Applizieren in der Schichttechnik sind nicht notwendig. Im Hinblick auf diese vorteilhaften Eigenschaften der Glasionomerzemente werden sie im breiten Umfang in medizinischen und zahnmedizinischen Bereichen eingesetzt, wie beispielsweise in DE 4023744 oder EP 386525 beschrieben. Glasionomerzemente können in der Medizin, z.B. als Knochenersatzmaterial, und in der Zahnheilkunde als Befestigungszemente, Unterfüllungs-, oder Fissurenversiegelungsmaterial, als Aufbau- oder als Füllungsmaterial verwendet werden. Dentale Glasionomerzemente werden beispielsweise in DE 3628822, DE 3941629, EP 694298 oder im Buch "Glasionomerzement" von A. D. Wilson und J. W. McLean, Quintessenz Verlags-GmbH 1988, beschrieben.

Nachteile der Glasionomerzemente, auch in Hinsicht als Amalgamersatz, sind neben einer unzureichenden Abrasionsresistenz, geringen Festigkeit, Sprödigkeit auch eine aufwendige Anmischung und eine schlechte Verarbeitbarkeit. Um diese Nachteile zu beseitigen sind vielfache Verbesserungen in der Zementbeschaffenheit vorgenommen worden.

Nach dem Stand der Technik wird zur Erhöhung der Belastbarkeit der Zemente ein möglichst hoher Füllstoffgehalt angestrebt. Damit ist auch eine erwünschte Erhöhung der Pastenkonsistenz der frisch angemischten Pulver-Flüssigkeits-Mischungen verbunden. Als Glasionomerzemente sind diese Zemente unter dem Namen "Hochviskose Glasionomerzemente" auf dem Markt. Ein Problem der hohen Pastenviskosität ist die dadurch erschwerte Anmischung von Pulver und Flüssigkeit. Die Anmischung dauert zu lange und führt bei der Anmischung per Hand aufgrund des hohen Pulver-Flüssigkeitsverhältnisses zu Dosier-Ungenauigkeiten. Als Lösung dieser Teilproblematik der Glasionomerzemente werden im Stand der Technik zwei Wege beschrieben. Zum einen die maschinelle Anmischung über Kapseln und zum anderen die Oberflächenbehandlung der Zementbestandteile.

Die dentalen wasserbasierenden Zemente werden dem Praktiker als Zweikomponentensysteme, zumeist bestehend aus Pulver und Flüssigkeit, angeboten. Neben der Möglichkeit der Handanmischung stehen dem Anwender Misch- oder Misch- und Applikationskapseln zur Verfügung, in denen die Zementbestandteile vorportioniert vorliegen, wie es beispielsweise in DE 19906887 dargelegt ist. Der Vorteil der Kapselanmischung ist, dass die maschinell vordosierten Zementbestandteile durch einen höheren Energieeintrag schneller und besser durchmischt werden und damit auch höhere Konsistenzen mischbar sind. Mittels Kapselmischgeräten werden die Zementbestandteile in der Kapsel vermischt und können anschließend aus der Kapsel entnommen werden oder bei Applikationskapseln direkt an den Behandlungsort appliziert werden. Letzteres macht es erforderlich, dass die Zemente aus der Kapsel über eine mitunter gering dimensionierte Kanüle ausdrückbar sein sollten. Zugleich sollten die dentalen Zemente für den Zahnarzt in gewohnter Weise zu verarbeiten sein, d.h. sie sollten plastisch verformbar, stopfbar und/oder modellierbar sein. Dafür müssen die Zemente jedoch über eine hohe Viskosität verfügen, was einer guten Ausdrückbarkeit aus einer dünnen Kanüle entgegensteht. Zur Problemlösung wird vorgeschlagen, die Kapselgeometrie, insbesondere die Ausbringkanülen, zu optimieren, wie es z.B. in DE 19906887 offenbart ist.

Durch die maschinelle Anmischung in der Kapsel kommt es durch den hohen Energieeintrag auch zu einer Erwärmung des Zementes, was wiederum zu einer unerwünschten Verkürzung der Verarbeitungszeit führt. Als Lösung dieses Problems wird die Oberflächenbehandlung der Pulverkomponenten entsprechend DE 3804469 vorgeschlagen. In DE 3804469 wird zur Verbesserung der Fließfähigkeit und der Mischbarkeit von Glasionomerzementen das Glaspulver auf der Oberfläche mit einer Säure und/oder einem Fluorid behandelt. Als zur Oberflächenbehandlung verwendete Säuren sind niedermolekulare Säuren wie Salzsäure, Phosphorsäure, Weinsäure, Zitronensäure, Glutarsäure, Äpfelsäure und Essigsäure genannt. Diese Oberflächenbehandlung, auch als Säurewaschung bekannt, führt zu einer Verlängerung der Verarbeitungszeit, hat jedoch keinerlei Auswirkungen auf die Verringerung der Klebrigkeit und Verbesserung der Ausdrückbarkeit des Zementes.

Es wurden durch Oberflächenbeschichtungen zahlreiche Versuche unternommen insbesondere die physikalische Beständigkeit, die Verarbeitbarkeit und die Feuchtigkeitsbeständigkeit von Glasionomerzementen zu verbessern, wie z.B. in DE 3628822.

Um die Endfestigkeit des ausgehärteten Zementes zu verbessern wird in DE 3941629 vorgeschlagen, die Fluoraluminiumsilikatgläser mit einer organischen Verbindung, die eine polymerisierbare Doppelbindung enthält, zu beschichten. Bevorzugt wird ein Silankupplungsmittel als Oberflächenbeschichtung eingesetzt.

Um eine Reizung der Zahnpulpa durch Dentalzemente, hier insbesondere Carboxylatzemente, zu verringern wird in DE 3915592 vorgeschlagen dem Zement eine Härtungsflüssigkeit, ein Härtungsbeschleuniger und ein Härtungsregler zuzusetzen. Die Härtungsflüssigkeit besteht aus einem mit Carboxylgruppen modifizierten Silikonöl, der Härtungsbeschleuniger aus Metalloxiden und/oder -hydroxiden und der Härtungsregler aus einer organischen oder anorganischen Säure. Als Letztere sind beispielhaft nur niedermolekulare Säuren, wie Essigsäure, Milchsäure, Äpfelsäure, Maleinsäure, Fumarsäure bzw. Salzsäure, Salpetersäure und Phosphorsäure genannt. Die Fließfähigkeit, die Löslichkeit, die Verarbeitungs- und Abbindezeit des Zementes werden als zufriedenstellend bezeichnet.

Weiteres bestehendes Problem der wasserbasierenden Zemente ist deren Feuchtigkeitsempfindlichkeit. Neben der Lösung des Problems durch aufwendige Verpackungen wird in DE 19914975 die Oberflächenbehandlung bzw. Oberflächenbeschichtung der Zementbestandteile vorgeschlagen. Nächstliegender Stand der Technik ist DE 19914975. In DE 19914975 wird zur Verbesserung der Feuchtigkeitsbeständigkeit dentaler Zemente vorgeschlagen einen Teil der zementbildenden Reaktionspartner mit einem organischen Oberflächenbeschichtungsmittel zu überziehen ohne dass dabei die mechanischen Werte wie Druck- oder Biegefestigkeit wesentlich verringert werden. Als Oberflächenbeschichtungsmittel werden dabei Mittel genannt, die auch bei der Tablettenherstellung bekannten sind. Als besonders geeignete Mittel werden Polymere mit Aminogruppen oder polymere Materialien auf Polysacharidbasis dargestellt. Zur Bereitstellung des dentalen Zementes müssen die zu beschichtenden Bestandteile einem aufwendigen Beschichtungsverfahren unterzogen werden.

Nachteilig wirkt sich bei den aus dem Stand der Technik hergestellten Zementen aus, auch wenn die wasserbasierenden Zemente verschiedene Verbesserungen erfahren haben, dass die Verbesserung einer Eigenschaft zumeist mit der Verschlechterung einer anderen Eigenschaft erkauft wird. Die Nachteile in der Fließfähigkeit, der Klebrigkeit, der schwierigen Anmischbarkeit und der mangelnden Ausdrückbarkeit aus einer Applikationskapsel bei gleichzeitig guter Verarbeitbarkeit werden durch die aus dem Stand der Technik bekannten Zemente nicht behoben. Das Ziel in den zitierten Druckschriften lag zudem nicht in der Verbesserung der Klebrigkeit, Ausdrückbarkeit und gleichzeitig guter Verarbeitbarkeit. Zudem wird dem Fachmann im Stand der Technik zur Verbesserung der Zementeigenschaften nur die Oberflächenbeschichtung bzw. Oberflächenbehandlung nahegelegt. Dies hat den Nachteil einer aufwendigen Herstellungsprozedur der Zementbestandteile zur Beschichtung. Dabei gilt es auch eventuelle Vorsichtsmaßnahmen im Umgang mit organischen Lösungsmitteln zu berücksichtigen.

Aufgabe der vorliegenden Erfindung ist es daher einen wasserbasierenden Glasionomer- Zement zu schaffen, der die vorstehend genannten Nachteile wasserbasierender Zemente nicht aufweist und der verbesserte Eigenschaften, insbesondere in der Anmischbarkeit, in der Lagerstabilität, in der Feuchtigkeitsempfindlichkeit, in der Ausdrückbarkeit aus einer Applikationskapsel sowie einer während der gesamten Verarbeitungszeit nicht vorhandenen Oberflächenklebrigkeit der angemischten Zementpaste am Applikationsinstrument aufweist. Gleichzeitig sollen die vorteilhaften Eigenschaften der ausgehärteten wasserbasierenden Zemente, wie beispielsweise die Druckhärte, die Löslichkeit, die Dentinhaftung, die Fluoridangabe und die biologische Verträglichkeit, nicht wesentlich verschlechtert werden. Aufgabe der vorliegenden Erfindung ist es daher auch einen wasserbasierenden Zement mit einer Pastenkonsistenz zur Verfügung zu stellen, die eine plastische Verformbarkeit des Zementes sowie eine Applikation mit üblichen Geräten zum Kondensieren, Stopfen von Amalgam in eine Zahnkavität erlaubt.

Gelöst wird die Aufgabe durch einen wasserbasierende Glasionomer-Zement, der 0,01 bis 10 Gew.% einer bei Raumtemperatur flüssigen aliphatischen Monocarbonsäure mit mehr als 5 C-atomen oder einer bei Raumtemperatur flüssigen Mischung aus aliphatischen Monocarbonsäuren mit mehr als 5 C-atomen umfaßt. Insbesondere wird die Aufgabe durch einen Glasionomerzement gelöst, der 0,5 bis 3,0 Gew.% Ölsäure beinhaltet.

Erfindungsgemäß eingesetzte Monocarbonsäuren mit mehr als 5 C-atomen weisen eine geringe bis keine Wasserlöslichkeit auf. Sie sind daher in üblichen wäßrigen Anmischflüssigkeiten der Zemente nicht löslich. Sie werden daher bevorzugt den Pulverkomponenten des Zementes zugesetzt. Überraschenderweise zeigte sich, dass sich die flüssigen aliphatischen Monocarbonsäuren durch einfache Zugabe und Mischen fein verteilen lassen. Überraschend ist auch, dass durch den Zusatz der flüssigen aliphatischen Monocarbonsäure zum pulverförmigen Zementbestandteil auch dessen Rieselfähigkeit bestehen bleibt, was einen nicht erwarteten Vorteil bei der Herstellung und Abfüllung der pulverförmigen Zementbestandteile darstellt. Trotz der damit verbundenen Hydrophobierung der Pulveroberfläche zeigt sich eine deutlich leichtere und schnellere Mischbarkeit der Pulver-Flüssigkeits-Komponenten. Der erhaltene Zement weist eine Pastenkonsistenz auf, die geschmeidiger und trotzdem weniger klebrig ist als ohne den Zusatz der erfindungsgemäß eingesetzten Monocarbonsäuren. Durch den erfindungsgemäßen Zement ist eine leichtere Ausdrückbarkeit aus einer Applikationskapsel bei gleichzeitig erhöhtem Füllstoffanteil möglich. D.h. auch, dass dadurch die Festigkeit des Zementes erhöht werden kann.

Überraschenderweise zeigt sich, dass trotz der geringen Klebrigkeit des Zementes am Instrument die Haftung am Zahn vor, während und nach der Zementaushärtung nicht negativ beeinflußt wird. Auch andere für die Beständigkeit des Zementes entscheidende physikalische Eigenschaften, wie Druckhärte, Löslichkeit und Abrasionsbeständigkeit werden nicht wesentlich beeinträchtigt.
Weiterhin ist der mit einer aliphatischen Monocarbonsäure versetzte Zement weniger feuchtigkeitsempfindlich und daher auch lagerstabil.

Als erfindungsgemäß geeignete aliphatische Monocarbonsäuren kommen alle bei Raumtemperatur flüssigen Säuren mit mehr als 5 C-atomen, insbesondere die sogenannten ungesättigten Fettsäuren, in Frage. Als besonders bevorzugt sind die folgenden Monocarbonsäuren anzusehen:
Capronsäure, Önanthsäure Caprylsäure, Caprinsäure, Pelargonsäure, Palmitoleinsäure, Ölsäure, Linolsäure, Linolensäure, Arachidonsäure und Clupanodonsäure.
Des weiteren sind bei Raumtemperatur flüssige Mischungen aus aliphatischen Monocarbonsäuren mit mehr als 5 C-atomen einsetzbar. Darunter fallen sowohl bei Raumtemperatur flüssige Mischungen aus mindestens einer bei Raumtemperatur flüssigen Monocarbonsäure mit mindestens einer bei Raumtemperatur festen Monocarbonsäure mit jeweils mehr als 5 C-atomen als auch bei Raumtemperatur flüssige Mischungen aus bei Raumtemperatur flüssigen Monocarbonsäuren mit mehr als 5 C-atomen.

Bei Raumtemperatur flüssige aliphatische Monocarbonsäuren mit mehr als 5 C-atomen oder bei Raumtemperatur flüssige Mischungen bewirken die vorteilhaften Effekte der verbesserten Ausdrückbarkeit, Verarbeitbarkeit und geringen Klebrigkeit des Zementes, da sie als Flüssigkeit im pulverförmigen Zementbestandteil fein verteilt werden können und aufgrund der Kohlenstoffkette mit mehr als 5 C-atomen einen aliphatischen Rest aufweisen, der einen hydrophoben Film auf dem frisch angemischten Zement ergibt. Bei aliphatischen Monocarbonsäuren mit 1 bis 5 C-atomen ist diese Hydrophobierung nicht zu beobachten. Di- oder Tricarbonsäuren haben ebenfalls nicht den gewünschten hydrophoben Effekt, zumal viele dieser Säuren bei Raumtemperatur nicht flüssig sind.

Im Gegensatz zu den in DE 3941629 eingesetzten Säuren sind die erfindungsgemäß eingesetzten Säuren nicht polymerisationsfähig. Weiterer Unterschied zu den als Beschichtungsmaterial im Stand der Technik eingesetzten Säuren ist, dass die erfindungsgemäßen Säuren keine Beschichtung darstellen, sondern fein verteilt im Zement vorliegen und nicht in aufwendigen Beschichtungsverfahren in den Zement eingebracht werden müssen. Die erfindungsgemäß eingesetzten Säuren reagieren während der Aushärtung mit den Glasbestandteilen unter Salzbildung und sind daher im ausgehärteten Zement fest eingebunden.

Die Einsatzmenge der erfindungsgemäß eingesetzten Säuren beschränkt sich auf einen maximalen Gewichtsanteil von 10 Gew.% bezogen auf die Gesamtzementmasse. Darüber hinaus führt der Zusatz an Säure zu einer Beeinträchtigung der Zementeigenschaften, z.B. in der Druckhärte.
Bevorzugt ist der Zusatz der bei Raumtemperatur flüssigen aliphatischen Monocarbonsäure bzw. deren Mischungen in einer Menge von 0,1 bis 5 Gew.%, besonders bevorzugt in einer Menge von 0,5 bis 3 Gew.%.

Die nachfolgenden Beispiele erläutern die Erfindung ohne einschränkend zu wirken.

### BEISPIELE - Kapselanmischung -

### Vergleichsbeispiel 1

Es wurde eine handelsübliche Misch- und Applikationskapsel des hochviskosen Glasionomer-Füllungszements VOCO lonofil Molar AC mit 460 mg statt wie üblich mit 430 mg der Pulverkomponente gefüllt, so dass sich eine erwünschte Erhöhung des Füllstoffanteils ergibt. Die Kapsel enthielt wie üblich ein mit 125 mg 35%iger Polyacrylsäurelösung gefülltes Aluminiumkissen, das die Flüssigkeit nach Aktivieren der Kapsel in die mit dem Pulver gefüllte Mischkammer der Kapsel injiziert. Die Kapsel wurde nach Herstellerangaben aktiviert und für 10 Sek. in einem geeigneten Kapselmischgerät gemischt, entsprechend DE 19906887. Der Zement ließ sich nur schwer mit einer dazugehörigen Applikationszange aus der Kanüle der geöffneten Kapsel herausdrücken. Die Zementoberfläche hatte eine etwas krümelige Struktur. Nach anfänglich guter Bearbeitbarkeit blieben geringe Rückstände an herkömmlichen zahnärztlichen Applikationsinstrumenten hängen, wodurch die Klebrigkeit am Instrument erhöht und damit die Applikation erschwert wurde.

### Beispiel 2

Es wurde die Pulverkomponente aus dem Vergleichsbeispiel 1 mit 0,5 Gew.% Ölsäure versetzt. Dazu wurden zu 25 g des Pulvers 125 mg Ölsäure gegeben und darin durch kräftiges Schütteln in einem verschlossenen Schraubdeckelglas verteilt. Die Mischung wurde durch eine 100 µm-Siebgaze vollständig gesiebt, wobei zurückgebliebene klumpige Siebrückstände auf dem Sieb verrieben wurden. Das Siebgut wurde nochmals homogen gemischt. Die erhaltene Mischung zeigte eine sehr gute Rieselfähigkeit, keine Separation oder Klumpenbildung und eine gute Lagerstabilität. 460 mg der Pulvermischung wurde wie im Vergleichsbeispiel 1 in eine mit gleichem Flüssigkeitskissen versehene Misch- und Applikationskapsel gefüllt. Die Kapsel wurde nach Herstellerangaben aktiviert und für 10 Sek. in einem geeigneten Kapselmischgerät gemischt. Der homogen angemischte Zement ließ sich sehr leicht mit einer dazugehörigen Applikationszange aus der Kanüle der geöffneten Kapsel herausdrücken. Er besaß sofort nach dem Ausdrücken eine geschmeidige homogene Konsistenz und zeigte während der gesamten Verarbeitungszeit keine Klebrigkeit an herkömmlichen zahnärztlichen Applikationsinstrumenten, d.h. diese ließen sich rückstandsfrei von der Oberfläche der Zementpaste entfernen.

Die notwendige Ausdrückkraft hängt eng mit der maximal aus der Kapsel ausbringbaren Zementmenge zusammen, da beide von der Fließfähigkeit der Zementpaste in und durch die Applikationskanüle abhängen. Aus der Tabelle 1 läßt sich erkennen, dass schon ein geringer Anteil von 0,5 % an Ölsäure einen deutlichen Effekt auf die Applizierbarkeit und die verfügbare Zementmenge hat. Es wurden in diesem Zusammenhang weitere Zemente entsprechend Beispiel 2 hergestellt und deren Applizierbarkeit untersucht.

**Tab. 1**

| Applizierbare Zementmenge des erfindungsgemäßen Beispiels 2 im Vergleich zum Beispiel 1 (Standardabweichung in Klammern) | | |
|---|---|---|
| | **Ölsäuregehalt (Gew.%)** | **Ausdrückbare Zementmenge (mg)** |
| Vergleichsbeispiel 1 | 0 | 280 (46) |
| Beispiel 2 | 0,5 | 395 (17) |
| Beispiel 3 | 5 | 388 (21) |

### Beispiele - Handanmischung -

### Vergleichsbeispiel 4

Es wurden die Pulver- und Flüssigkeitskomponenten eines handelsüblichen, hochviskosen Glasionomerzementes eingesetzt (VOCO lonofil Molar, VOCO GmbH). Das Pulver besteht aus einem Aluminiumfluorosilikatglas, gefriergetrockneter Polyacrylsäure, Weinsäure und Farbstoffpigmenten. Die Flüssigkeit besteht aus einer wäßrigen Polyacrylsäurelösung. Pulver und Flüssigkeit wurden nach Herstellerangaben von Hand gemischt.

### Beispiele 5 bis 11

Jeweils 100 g Pulver aus Vergleichsbeispiel 4 wurde mit der in Tabelle 2 angegebenen Menge Ölsäure bzw. Caprylsäure zwischen 0,5 Gew.% und 4 Gew.% versetzt und in einem geschlossenen Behälter durch kräftiges Schütteln gemischt. Die Mischung wurde durch eine 100µm-Siebgaze gesiebt, wobei zurückgebliebene klumpige Siebrückstände auf dem Sieb verrieben wurden. Das Siebgut wurde nochmals homogen gemischt. Die erhaltenen Mischungen zeigten eine gute Rieselfähigkeit, keine Separation oder Klumpenbildung und eine gute Lagerstabilität. Pulver und Flüssigkeit wurden wie im Vergleichsbeispiel 4 nach Herstellerangaben von Hand gemischt.
Mit zunehmender Öl- bzw. Caprylsäuremenge ließ sich das Pulver leichter und schneller in die Flüssigkeit einarbeiten. Die Pastenkonsistenz war geschmeidiger und ließ sich aufgrund der geringen Klebrigkeit sehr gut verarbeiten.
Um die Mischbarkeit der mit Öl- bzw. Caprylsäure versetzten Zemente zu untersuchen, wurden die notwendigen Anmischzeiten zum Erreichen einer homogenen Paste beginnend mit dem ersten Kontakt des Pulvers mit der Flüssigkeit gemessen. Dazu wurden jeweils 400 mg Pulver und 100 mg Flüssigkeit auf einer Anmischglasplatte abgewogen und mit einem Kunststoffspatel gemischt. Die ermittelten Zeiten sind in Tabelle 2 zusammengefaßt. Um zu ermitteln, ob durch die Zugabe der Öl- bzw. Caprylsäure einen Einfluß auf die Druckhärte ausgeübt wird, wurde entsprechend ISO 9917 die Druckhärte der nach Beispiel 4 bis 11 hergestellten Zemente untersucht.

**Tabelle 2**

| Einfluß des Öl- bzw. Caprylsäuregehaltes auf die Anmischzeit und Druckhärte der Zemente nach Beispiel 4 bis 11 | | | | | | | | |
|---|---|---|---|---|---|---|---|---|
| **Beispiel** | **4 (Vergleich)** | **5** | **6** | **7** | **8** | **9** | **10** | **11** |
| Ölsäureanteil (Gew.%) | 0 | 0,5 | 1 | 2 | 4 | 0,2 | - | - |
| Caprylsäureanteil (Gew.%) | 0 | - | - | - | - | 0,5 | 0,5 | 2 |
| Anmischzeit (Sek.) | 50 | 35 | 30 | 35 | 30 | 33 | 30 | 32 |
| Druckhärte (MPa) | 189 | 198 | 192 | 176 | 161 | 195 | 184 | 173 |

Die Ergebnisse veranschaulichen, dass durch eine erfindungsgemäße Zugabe von Ölsäure, Caprylsäure oder deren Mischung deutlich kürzere Mischzeiten notwendig sind, was trotz Hydrophobierung des Pulvers durch die Monocarbonsäuren auf eine leichtere Mischbarkeit zurückzuführen ist. Die Druckhärte des Zementes wird durch den Zusatz der Monocarbonsäuren nur wenig beeinflußt und liegt selbst bei einem Anteil von 4 Gew.% Ölsäure noch über dem in der Norm ISO 9917 geforderten Mindestwert von 130 MPa.

### Beispiel 12

Um die Auswirkungen der Monocarbonsäurezusätze auf die physikalischen Eigenschaften wasserbasierender Zemente zu untersuchen, wurde entsprechend Vergleichsbeispiel 1 bzw. 4 handelsübliche Zemente mit den in Tabelle 3 angegebenen Mengen an Monocarbonsäure versetzt. Es wurde anschließend die Druckhärte nach ISO 9917 (1991), die Löslichkeit nach ISO 7489 (1986) und die Dentinhaftung nach ISO TR 11405 (1994) der ausgehärteten Zemente untersucht. Die ermittelten Ergebnisse sind in Tabelle 3 aufgeführt.

**Tabelle 3**

| Auswirkungen der Monocarbonsäurezusätze auf die physikalischen Eigenschaften wasser-basierender Glasionomer- Zemente (Druckhärte nach ISO 9917 (1991), Löslichkeit nach ISO 7489 (1986), Dentinhaftung nach ISO TR 11405 (1994)) | | | | | | |
|---|---|---|---|---|---|---|
| **Zement- name (Hersteller)** | **Zement** | **Ölsäureanteil (Gew.%)** | **Caprylsäureanteil** (Gew.%) | **Druckhärte (MPa)** | **Löslichkeit (Gew.%)** | **Dentinhaftung (MPa)** |
| Ketac Molar (Espe) | Hochviskoser Glasionomerzement | 0 | 0 | 215 | 0,05 | 4,5 |
| Ketac Molar (Espe) | Hochviskoser Glasionomerzement | 2 | 0 | 190 | 0,01 | 4,3 |
| Ketac Cem (Espe) | Befestigungs-Glasionomerzement | 0 | 0 | 120 | 0,11 | 3,8 |
| Ketac Cem (Espe) | Befestigungs-Glasionomerzement | 2 | 0 | 120 | 0,15 | 2,9 |
| Ketac Cem (Espe) | Befestigungs-Glasionomerzement | 0 | 1 | 122 | 0,11 | 3,0 |

Die Ergebnisse der Untersuchungen zeigen, dass der erfindungsgemäße Zusatz an Monocarbonsäure die physikalischen Eigenschaften wasserbasierende Glasionomer-Zemente nicht negativ bzw. in bezug zur Löslichkeit sogar positiv beeinflußt. Die in den Normen Druckhärte ISO 9917 (1991), Löslichkeit ISO 7489 (1986), Dentinhaftung ISO TR 11405 (1994) festgelegten Mindestanforderungen werden immer übertroffen.

Die Beispiele belegen, dass der erfindungsgemäße Zement verbesserte Eigenschaften, insbesondere in der Anmischbarkeit, in der Lagerstabilität, in der Feuchtigkeitsempfindlichkeit, in der Ausdrückbarkeit aus einer Applikationskapsel sowie einer während der gesamten Verarbeitungszeit nicht vorhandenen Oberflächenklebrigkeit der angemischten Zementpaste am Applikationsinstrument aufweist. Gleichzeitig werden die vorteilhaften Eigenschaften der ausgehärteten wasserbasierenden Glasionomer-Zemente, wie beispielsweise die Druckhärte, die Löslichkeit und die Dentinhaftung nicht verschlechtert.

## Patentansprüche

1. Glasionomerzement umfassend 0,01 bis 10 Gew. % einer bei Raumtemperatur flüssigen aliphatischen Monocarbonsäure mit mehr als 5 C-Atomen oder einer bei Raumtemperatur flüssigen Mischung aus aliphatischen Monocarbonsäuren mit mehr als 5-C-Atomen.

2. Glasionomerzement nach Anspruch 1, **dadurch gekennzeichnet, dass** die Monocarbonsäure oder die Mischung der Monocarbonsäuren in einer Menge von 0,1 bis 5 Gew. % enthalten ist.

3. Glasionomerzement nach Anspruch 2, **dadurch gekennzeichnet, dass** die Monocarbonsäure oder die Mischung der Monocarbonsäuren in einer Menge von 0,5 bis 3 Gew. % enthalten ist.

4. Glasionomerzement nach Anspruch 1, 2, oder 3, **dadurch gekennzeichnet, dass** die Monocarbonsäure Ölsäure ist.

5. Glasionomerzement nach einem der Ansprüche 1 bis 4 zur Verwendung als Dentalmaterial in der Zahnheilkunde.

6. Glasionomerzement nach einem der Ansprüche 1 bis 4 zur Verwendung als Material in der Medizin.

## Claims

1. Glass ionomer cement, comprising 0.01 to 10% by weight of an aliphatic monocarboxylic acid with more than 5 carbon atoms which is liquid at ambient temperature or of a mixture of aliphatic monocarboxylic acids with more than 5 carbon atoms which is liquid at ambient temperature.

2. Glass ionomer cement according to Claim 1, **characterized in that** the monocarboxylic acid or the mixture of monocarboxylic acids is present in an amount of 0.1 to 5% by weight.

3. Glass ionomer cement according to Claim 2, **characterized in that** the monocarboxylic acid or the mixture of monocarboxylic acids is present in an amount of 0.5 to 3% by weight.

4. Glass ionomer cement according to Claim 1, 2 or 3, **characterized in that** the monocarboxylic acid is oleic acid.

5. Glass ionomer cement according to one of Claims 1 to 4, for use as dental material in dentistry.

6. Glass ionomer cement according to one of Claims 1 to 4, for use as material in medicine.

## Revendications

1. Ciment verre ionomère comprenant 0,01 à 10% en poids d'un acide monocarboxylique aliphatique liquide à température ambiante présentant plus de 5 atomes de carbone ou d'un mélange liquide à température ambiante d'acides monocarboxyliques aliphatiques présentant plus de 5 atomes de carbone.

2. Ciment verre ionomère selon la revendication 1, **caractérisé en ce que** l'acide monocarboxylique ou le mélange des acides monocarboxyliques est contenu en une quantité de 0,1 à 5% en poids.

3. Ciment verre ionomère selon la revendication 2, **caractérisé en ce que** l'acide monocarboxylique ou le mélange des acides monocarboxyliques est contenu en une quantité de 0,5 à 3% en poids.

4. Ciment verre ionomère selon la revendication 1, 2 ou 3, **caractérisé en ce que** l'acide monocarboxylique est l'acide oléique.

5. Ciment verre ionomère selon l'une quelconque des revendications 1 à 4 destiné à une utilisation comme matériau dentaire en médecine dentaire.

6. Ciment verre ionomère selon l'une quelconque des revendications 1 à 4 destiné à une utilisation comme matériau en médecine.
